(19)

**Europäisches Patentamt**
**European Patent Office**
**Office européen des brevets**

(11) **EP 4 124 844 A1**

(12) # EUROPÄISCHE PATENTANMELDUNG

(43) Veröffentlichungstag:
**01.02.2023 Patentblatt 2023/05**

(21) Anmeldenummer: **21000204.4**

(22) Anmeldetag: **26.07.2021**

(51) Internationale Patentklassifikation (IPC):
**G01N 3/04** *(2006.01)* **G01N 3/06** *(2006.01)*
**G01N 3/12** *(2006.01)* **G01N 33/02** *(2006.01)*
**G01N 3/20** *(2006.01)* **G01M 5/00** *(2006.01)*

(52) Gemeinsame Patentklassifikation (CPC):
**G01N 3/04; G01N 3/068; G01N 3/20; G01N 33/02;**
G01N 2203/0023; G01N 2203/0044;
G01N 2203/0067; G01N 2203/0075;
G01N 2203/0218; G01N 2203/0252;
G01N 2203/0282; G01N 2203/0411;
G01N 2203/0423; G01N 2203/0682

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Universitatea Stefan cel Mare Suceava - Romania**
**720229 Suceava (RO)**

(72) Erfinder:
- **Gutt, Gheorghe**
  **Sat Sf. Ilie - Scheia (RO)**
- **Amariei, Sonia**
  **Sat Sf. Ilie - Scheia (RO)**

(54) **VERFAHREN UND VORRICHTUNG ZUR BESTIMMUNG VON MATERIAL KENNWERTEN FÜR ESSBARE MEMBRANEN**

(57) Die Erfindung betrifft ein Verfahren und eine Vorrichtung zur Bestimmung des Berstwiederstandes $\sigma_m$, des spezifischen Elastizitätsmoduls $E_{ms}$ und der spezifischen mechanischen Arbeit $W_{ms}$, alle drei Materialkennwerte werden zur Charakterisierung von Proben (**1**) aus essbaren Membranen, die ihrerseits für Verpackung von Lebensmitteln verwendet werden. Gemäss dem Verfahren wird auf die Oberfläche der zu testenden Membran linear ein steigender Luftdruck aufgebracht es wird der auf die Membran ausgeübte Druck $p_m$ und die Höhe $h_m$ der Kugelkalotte, die aus der Verformung der Membran infolge des Druckanstiegs resultieren, gemessen und gespeichert. Über eine Software werden die Berstfestigkeit, der spezifische Elastizitätsmodul und die spezifischen mechanischen Arbeit berechnet. Die erfindungsgemäße Vorrichtung enthält die konstruktiven Elemente, die das Verfahren zur Messung der oben genannten charakteristischen Kennwerte verwirklicht.

*FIG.4*

Processed by Luminess, 75001 PARIS (FR)

**Beschreibung**

Das Verfahren und die Vorrichtung gemäß der Erfindung stellen ein Mittel zur Bestimmung der Berstfestigkeit des spezifischen Elastizitätsmoduls und der spezifischen mechanischen Verformungsarbeit von essbaren Membranen dar.

Essbare Membranen sind für die Verpackung verschiedener Lebensmittel bestimmt und werden zusammen mit den verpackten Lebensmitteln verzehrt. Diese Membranen stellen moderne Verpackungen dar, die Teil der zyklischen Wirtschaft sind, die generisch definiert wird durch: "Nichts wird verbrannt, nichts wird begraben, alles wird wiederverwendet" und sind natürliche Polymerstrukturen, die aus Lebensmitteln gewonnen werden, die zusätzlich zu der Grundfunktion jeder Verpackung durch Zusatzstoffe dem Körper auch wichtige Antioxidantien sichern können. Das Verhalten dieser Membranen bei mechanischer Belastung unterscheidet sich von den ungenießbaren Membranen, aus synthetischen Polymeren, die heute noch in großem Umfang für Lebensmittelverpackungen verwendet werden, entsprechend erfordern die essbaren Membranen spezielle Testverfahren und Vorrichtungen.

Die Erfindung basiert auf einem Verfahren und einer Vorrichtung , die als Hauptmittel zur Bestimmung der Berstfestigkeit von essbaren Membranen verwendet wird. Das dem Verfahren zugrunde liegende Prinzip und die dazugehörige Vorrichtung erlauben neben der Bestimmung des Werts der Berstfestigkeit auch des Wertes des spezifischen Elastizitätsmoduls und des Werts der spezifischen mechanischen Verformungsarbeit. Diese Kennwerte sind wertvolle Mittel zur Bestimmung des Verhaltens von essbaren Membranen bei mechanischen Belastungen und werden zu derer fortgeschrittenen Charakterisierung beim Verpacken, Transport und Verzehr verwendet.

Zum Stand der Technik, bezüglich der Prüfung der Berstfestigkeit von dünnen Verpackungsmaterialien wie synthetische Polymerfolien und Papier, sind den Autoren zwei Dokumente bekannt.

Das Dokument [D1], Standard ASTM D1709 - 03, beschreibt ein Verfahren und eine Vorrichtung , die auf der Messung der Fallenergie basiert, augedrückt in Masse Einheiten eines fallenden Körpers, der eine halbkugelförmige Geometrie an der Unterseite hat, Energie die nötig ist um 51% der getesteten Proben eines Polymerfilms, durch Rissbildung oder durch Bersten zu zerstören .

Zur Prüfung der Berstfestigkeit von Papier ist auch das im Dokument [D2], Norm DIN EN ISO 2758, beschriebene Verfahren bekannt, bei dem eine Probe mit steigendem Druck gleichmäßig belastet wird bis diese zum Riss oder zum Bersten kommt. Die Berstfestigkeit wird in Kilopascal ausgedrückt. Das Verhältnis zwischen dem Wert der Berstfestigkeit und der Masse einer Papierprobe, ausgedrückt in $g/m^2$, definiert den Berstindex.

Das durch die Erfindung gelöste Problem besteht in einem entwickelten und definierten Verfahren zur Bestimmung der Berstfestigkeit, des spezifischen Elastizitätsmoduls und der spezifischen mechanischen Verformungsarbeit sowie in einer Vorrichtung mit der kurzzeitig die oben genannten Material Kennwerte, an Proben aus essbaren Membranen bestimmt werden.

Durch das Verfahren und der Vorrichtung wird der Vorteil einer schnellen und fortgeschrittene Charakterisierung, durch Bestimmung der Berstfestigkeit, des spezifischen Elastizitätsmoduls und der spezifischen mechanischen Verformungsarbeit, ein Mass des Verhaltens essbarer Membranen bei mechanischer Belastung, erreicht.

Das erfindungsgemäße Verfahren besteht darin, einen ansteigenden und bekannten linearen Luftdruck auf eine Probe in Form einer Scheibe mit einem Durchmesser von 50 mm, hergestellt aus einer essbaren zu testenden Membran, aufzubringen. Die Membranprobe wird zwischen zwei Edelstahlringen und zwei Dichtungen aus Silikongummi am Oberteil eines zylindrischen Edelstahlbehälters abgedichtet. Das Innere des Edelstahlbehälters wird durch einen zunehmenden linearen Luftdruck versorgt der seinerseits durch einen programmierbaren elektronischen Druckregler gesichert wird. Bei steigendem Luftdruck verform sich die Probe der getesteten Membran mit der Geometrie eines wachsendes Kugelsegments, bis das Membranmaterial platzt, daß zu einem plötzlichen Druckabfall im Inneren des zylindrischen Edelstahlbehälters führt.

Während des Aufbringens des linear ansteigenden Drucks bis zum Abfall auf Null infolge der Zerstörung der Membran wird der auf die Membran ausgeübter Druck und die Höhe der verformten Membran gemessen und elektronisch gespeichert. Die Druckwerte werden mit einem elektronischen Manometer gemessen dass auch über die Funktion der automatischen Speicherung des Spitzenwertes des maximalen Drucks beim Bersten der Membran (Berstfestigkeit), über eine USB-Schnittstelle und einen Temperatursenzor zur Messung der Lufttemperatur im Inneren des Edelstahlbehälters, verfügt. Der maximale Druckwert beim Bersten der Membran stellt den Berstwiderstand, ausgedrückt in Mpa, dar.

Der Höhenverlauf der Kugelkalotte der verformten Membran wird bis zum Berstmoment wird durch einen berührungslosen Wegsensor, basierend auf konfokaler Laser Triangulation, gesichert. Mit den Messwerten: Membrandruck - Höhe der Kugelkalotte wird der spezifische Elastizitätsmodul und der Wert der erforderlichen spezifischen mechanischen Verformungsarbeit, bis die Membran platzt, berechnet.

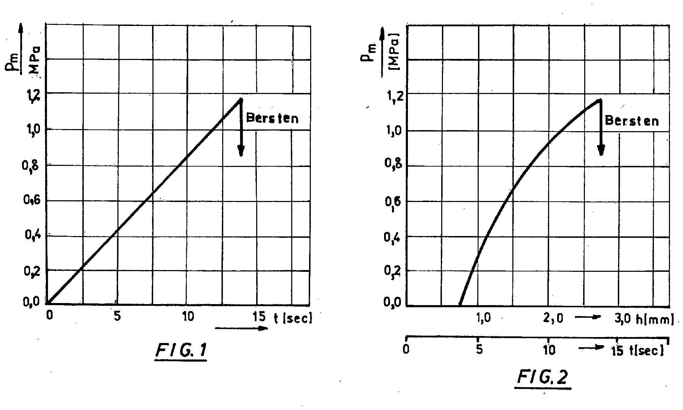

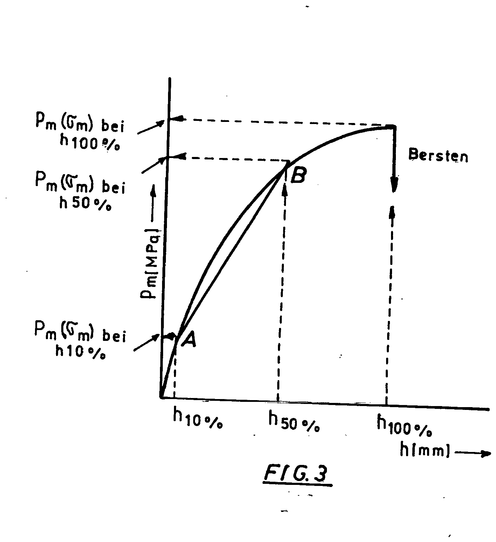

Im Folgenden wird das Verfahren, in Verbindung mit Fig.1, Fig.2 und Fig.3, beschrieben:

Abb.1 - Diagramm des auf die Membran augeüten Druckes als Zeit Funktion
Abb.2 - Diagramm der Höhenentwicklung der Kugelkalotte in Abhängigkeit des auf die Membranprobe ausgeübten Drucks

Abb.3 - Diagramm in Koordinaten Druck - Höhe der Kugelkalotte mit der Anzeige der Sekante, welche die Kurve beim Höhenwert $h_{10\%}$ und dem Höhenwert $h_{50\%}$ schneidet

Die Berstfestigkeit $\sigma_{pm}$ der geprüften Membran wird automatisch im Moment des Berstens ermittelt, und wird als Verhältnis zwischen dem maximalen Luftdruck $p_{max}$, der im zylindrischen Edelstahlgehäuse im Berstmoment gemessen wurde, und der Oberfläche Ao-der geprüften Membranoberfläche, ausgedrückt:

$$\sigma_{pm} = \frac{p_{\max}}{A_0} = \frac{p_{\max}}{\pi \cdot r^2} = 127{,}38 \cdot p_{\max} \qquad [\text{MPa}] \qquad (1)$$

Der Wert des maximalen Drucks wird vom elektronischen Manometer auf der Grundlage des Nullwerts der ersten Ableitung des Verhältnisses zwischen der Änderung des auf die Membran aufgebrachten Drucks $p_m$ und der Änderung $h_m$ der Höhe des gebildeten Kugelsegments der Membran, erfasst:

$$d\frac{\Delta p_m}{\Delta r} = 0 \qquad (2)$$

Der Elastizitätsmodul (Young-Modul) wird klassisch aus dem Zugversuch des geprüften Materials ermittelt und als Verhältnis zwischen der auf das Material aufgebrachten mechanischen Spannung und seinem Verformungsgrad ausgedrückt, wobei beide Größen nach dem Hookeschen Gesetz für die linear-elastische Verformung gemessen werden. In der Praxis folgen nur Stähle und teilweise Holz dem Hookeschen Gesetz, die anderen Materialien haben, vom Anfang der Belastung bis zum Bruch, einen gekrümmten Verlauf der Kurve mechanische Spannung $\sigma$ - Verformungsgrad $\varepsilon$. Für diese Materialien, zu denen auch die essbaren Membranen gehören, wird ein spezifischer Elastizitätsmodul definiert. Für eine scheibenförmige Membran, zunächst flach, die einen zylindrischen Raum verschließt, wird kontinuierlich Luft mit einem linear ansteigenden Druck $p_m$ eingeleitet, wobei sich die Membran zeitlich zu einem Kugelsegment mit der Höhe $h_m$ entwickelt,

Erfindungsgemäß wird der für essbare Membranen spezifische Elastizitätsmodul *Ems* aus dem Verhältnis zwischen dem auf die Membran ausgeübten Luftdruck $p_m$ und dem spezifischen Verformungsgrad $\varepsilon_s$ bestimmt. Dazu werden automatisch die Druckwerte $p_m$ erfasst und gespeichert, die vom Nullwert aufsteigend bis zum Bersten der Membran aufgebracht werden. Gleichzeitig mit den Druckwerten werden auch die Höhenwerte $h_m$ der Kugelkalotte erfasst und gespeichert. Aus den gespeicherten Werpaaren $p_m$ - hm wird automatisch die Belastungskurve sowie die Sekante, welche die Kurve bei $h_{10\%}$ und $h_{50\%}$ schneidet. generiert, unter diesen Bedingungen hat der spezifische Verformungsgrad $\varepsilon_s$ der Membran den Ausdruck:

$$\varepsilon_s = \frac{h_{50\%} - h_{10\%}}{h_{10\%}} \qquad (3)$$

dementsprechend wird der Elastizitätsmodul $E_{ms}$ automatisch durch die Beziehung:

$$E_{ms} = \frac{p_{m50\%} - p_{m10\%}}{\varepsilon_s} = \frac{(p_{m50\%} - p_{m10\%}) \cdot (h_{10\%})}{h_{50\%} - h_{10\%}} \qquad [Mpa] \qquad (4)$$

definiert.

Die zur Verformung eines Werkstoffes aufgewendete mechanische Arbeit *W* wird klassisch als Produkt aus der Kraft welche die Verformung verursacht und dem Verformungsweg definiert. Die spezifische mechanische Arbeit $W_{ms}$, für die Verformung einer Membran durch einen darauf aufgebrachten Luftdruck wird durch das Produkt zwischen dem auf die Membran gebrachten Druck $p_m$ und der Höhe $h_m$ der Membranverformung ausgedrückt:

$$W_{ms} = p_m.h_m \qquad [J] \qquad (5)$$

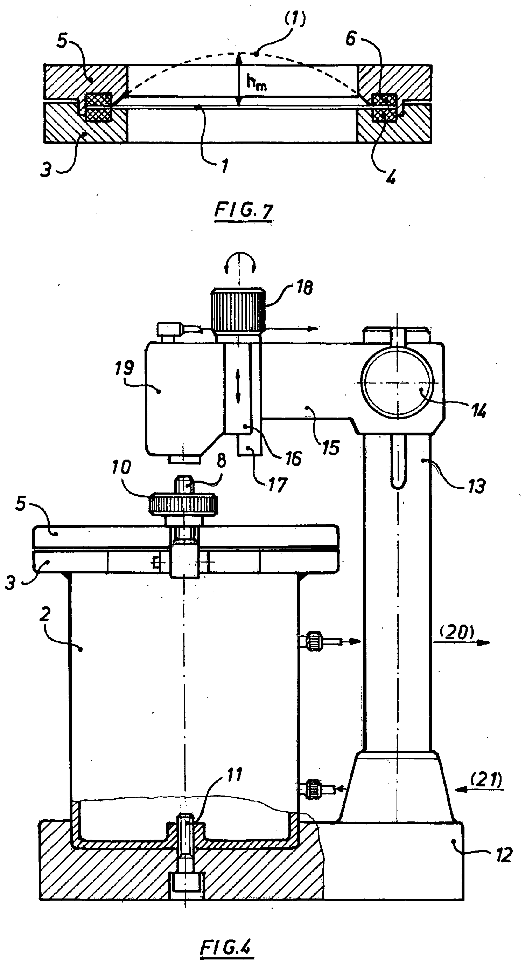

Im Folgenden, wird eine Ausführungsform der Vorrichtung zur Verwirklichung des Verfahrens in Verbindung mit Fig.4, Fig.5, Fig.6 und Fig.7, Fig.8, gezeigt:

Abb.4 - Seitenansicht der Vorrichtung

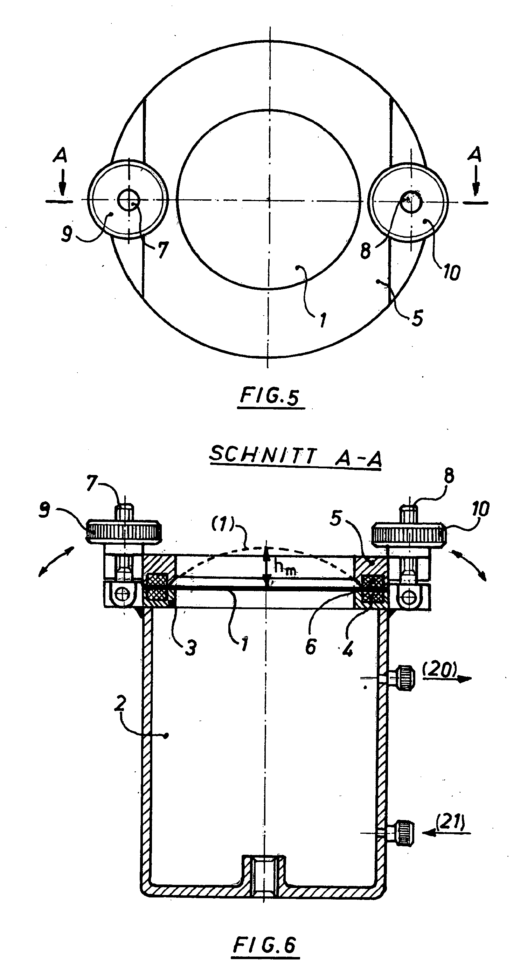

Abb.5 - Ansicht von oben auf den zylindrischen Edelstahlbehälter mit Anbauteilen
Abb.6 - Schnitt durch den zylindrischen Edelstahlbehälter mit Anbauteilen
Abb.7 - Ansicht der Verdichtung der Probe aus einer essbaren Membran

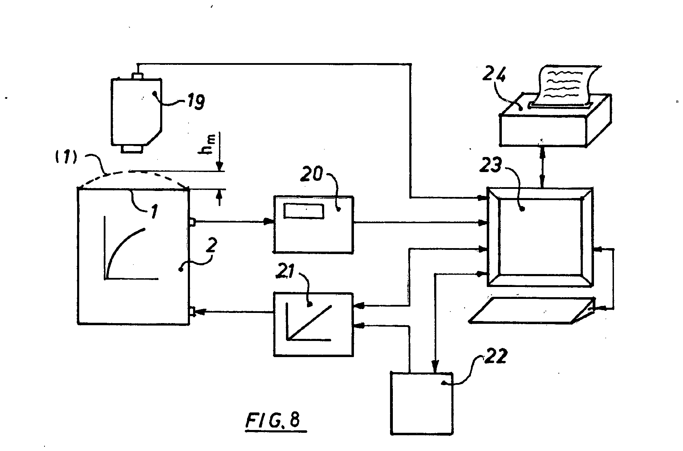

Abb.8 - Schematische Darstellung der Vorrichtung

Die Vorrichtung zur Prüfung einer Probe **1**, aus einer essbaren Membran besteht aus einem Edelstahlbehälter **2**, zwei Ringen **3** und **5** aus Edelstahl, zwei Dichtungen **4** und **6** aus Silikongummi, zwei Kippschrauben **7** und **8**, zwei Rändelmuttern **9** und **10**, einer Schraube **11**, einem Gusseisensupport **12**, einer zylindrische Stahlsäule **13**, einer Rändelschraube **14**, einer Stahltraverse **15** einer Schwalbenschwanz Führung **16** und **17**, einer Schraube **18** mit gerändeltem Kopf, einem berührungslosen Bewegungssensor **19** mit konfokaler Laser Triangulation, einem elektronisches Manometer **20** mit USB-Schnittstelle und Speicherung des maximalen Luftdruckwertes, einer pneumatische Einheit **21** zur linearen progressiven Erhöhung des Luftdrucks, einer pneumatische Einheit **22** zur Sicherung eines konstanten Drucks, einem Computer **23**, einem Drucker **24** und einer Software zur Programmierung der Druckerhöhung im Edelstahlbehälter **2** sowie zur Datenverarbeitung.

Die Arbeitsweise und die Schrittfolge des erfindungsgemäßen Verfahrens sind wie folgt:

a.- Zwecks der Prüfung wird eine Probe **1** unter Form einer Scheibe mit einem Durchmesser von 50 mm, aus einer essbaren Membran mit einer Handstanze gefertigt;

b.- Die gerändelten Muttern **9** und **10** werden gelöst und danach werden sie zusammen mit den Schrauben **7** und **8** um $180^0$ gekippt.

c.- Der Edelstahlring **5** wird entfernt und nacher wird die Membranprobe 1 zentrisch über die Silikongummidichtung **4** gelegt, danach wird der Edelstahlring **5** wieder in die Ausgangsposition gebracht, so dass die Probe zwischen der Silikongummidichtung **4** und dem Silikongummidichtung **6** Silikon liegt.

d.- Die kippbaren Schrauben **7** und **8** werden in die Ausgangsposition gebracht und die gerändelten Muttern **9** und **10** werden angezogen bis die Probe **1** gegen DrucKluftverlust abgesicher ist.

e.- Über den Computer **23** der Rändelschraube **14** und der Schwalbenschwanz Führung **16** und **17** wird der Nullwert des Drucks, der Nullwert der Membranverformung und der Wert der Aufbringungsgeschwindigkeit des Drucks auf die Membran der Probe **1** eingestellt.

f.- Durch den progressiv und linear wachsendem Druck im zylindrischen Edelstahlgefäß **2** wird die Membran der Probe **1** kontinuierlich belastet dass zu derer elastischen Verformung unter Form eines des Kugelsegments führt. Während des Verformungswegs des Kugelsegments werden gleichzeitig folgende Parameter gemessen, gespeichert und verarbeitet: der auf die Membran ausgeübte Druck $p_m$, die Höhe $h_m$ des Kugelsegments der verformten Membran die Lufttemperatur $T$ der innerhalb des zylindrischen Edelstahlgefäß **2**

g.- Nach dem erfindungsgemäßen Verfahren erfolgt im nächsten Schritt die Berechnung und Anzeige der Werte für: die Berstfestigkeit $\sigma_{pm}$, den spezifischen Elastizitätsmodul $E_{ms,}$ und die mechanische Verformungsarbeit $W_{ms}$, für die essbare Membran aus der Probe **1**.

Auf Wunsch können die Diagramme des Wachstumsverlaufs der Höhe des Kugelsegments als Funktion des aufgebrachten Drucks und das Diagramms des Verlaufs des aufgebrachten Drucks als Funktion der Zeit, in Echtzeit, angezeigt werden. Durch das Testen einer großen Charge von essbaren Membranproben **1** , mit unterschiedlichen Zusammensetzungen und unterschiedlichen Dicken werden Kurvenscharen erhalten, die für die erweiterte Charakterisierung und Verhalten bei unterschiedlicher mechanischer Belastungen verwendet werden.

**Patentansprüche**

1. Die Erfindung Verfahren und Vorrichtung zur Bestimmung von Materialkennwerten für essbare Membranen in derer Struktur ein elektronisches Manometer (**20**), ein elektronischen Wegsensor (**19**), ein Computer (**23**) und ein Drucker (**24**) verwendet wird, **dadurch gekennzeichnet, dass** erfindungsgemäß das Verfahren es ermöglicht, die Werte der Berstfestigkeit $\sigma_m$, des spezifischen Elastizitätsmoduls $E_{ms}$ und der spezifischen mechanischen Verformungsarbeit $W_{ms}$ für Proben (**1**) aus essbaren Membranen zu bestimmen und eine Vorrichtung, die erfindungsgemäß, das Verfahren verwirklicht.

2. Die Erfindung Verfahren und Vorrichtung zur Bestimmung von Materialkennwerten für essbare Membranen **dadurch gekennzeichnet, dass** für die Prüfungen Proben (1) verwendet werden, die aus essbaren Membranen, mit einem Durchmesser von 50 mm, gestanzt sind.

3. Verfahren zur Bestimmung der Berstfestigkeit nach Anspruch 1 von Proben (**1**) aus essbaren Membranen hergestellt, **dadurch gekennzeichnet, dass** die Berstfestigkeit $\sigma_m$ einer Probe (**1**) im Moment des Berstens hder Membran gegeben wird und durch das Verhältnis des maximalen Luftdrucks $p_{max}$, im Inneren eines zylindrischen Edelstahlbehälters (**2**), zur Fläche $A_0$ der Anfangsoberfläche von 0,00785 m² der Probe (**1**) ausgedrückt ist:

$$\sigma_{pm} = \frac{p_{\max}}{A_0} = \frac{p_{\max}}{\pi \cdot r^2} = 127{,}38 \cdot p_{\max} \qquad [MPa]$$

wobei das Mittel zur Messung des Höchstdrucks $p_{max}$ ein elektronisches Manometer (**11**) mit automatischer Speicherung des Höchstwertes des Luftdrucks zum Zeitpunkt des Berstens und ein Temperatursensor zur automatischen Messung der Lufttemperatur im Edelstahlbehälter (**2**), ist.

4. Verfahren zur Bestimmung des spezifischen Elastizitätsmoduls $E_{ms}$ des Materials essbarer Membranen nach Anspruch 1, **dadurch gekennzeichnet, dass** der spezifische Elastizitätsmodul $E_{ms}$ durch das Verhältnis aus dem Wert der Sekante zur Kurve $p_m$ - $h_m$, mit den Koordinaten der zwei Schnittpunkte der Kurve: $p_{10\%}$ und $h_{10\%}$ bzw. $p_{50\%}$ und $h_{50\%}$, und dem Wert der spezifischen Verformung gegeben wird, wobei die spezifische Verformung der Membran der Probe (**1**) folgenden Ausdruck hat:

$$\varepsilon_{ms} = \frac{h_{50\%} - h_{10\%}}{h_{10\%}}$$

entsprechend bekommt der spezifische Elastizitätsmodul $E_{ms}$ den Ausdruck:

$$E_{ms} = \frac{p_{m50\%} - p_{m10\%}}{\varepsilon} = \frac{(p_{m50\%} - p_{m10\%}) \cdot (h_{10\%})}{h_{50\%} - h_{10\%}} \qquad [Mpa]$$

wobei die Mittel zum Messen der Entwicklung des Drucks $p_m$ und der Entwicklung der Höhe $h_m$ des Radius der Kugelkalotte das elektronische Manometer (**20**) bzw. ein berührungsloser Sensor (**19**) sind.

5. Verfahren zur Bestimmung der spezifischen mechanischen Arbeit $W_{ms}$, nötig zur Verformung essbaren Membran aus der Probe (**1**), nach Anspruch 1, **dadurch gekennzeichnet, dass** der Wert der spezifischen mechanischen Arbeit $W_{ms}$ durch das Produkt zwischen dem Wert des auf die Membran ausgeübten Drucks $p_m$ und dem Wert der Höhe $h_m$ der Kugelkalotte gegeben wird:

$$W_{ms} = p_m \cdot h_m \qquad [J]$$

die Mittel zum Messen des Drucks $p_m$ und der der Höhe $h_m$ *des* Radius der Kugelkalotte sind das elektronische Manometer (**20**) und der berührungslose Sensor (**19**).

6. Vorrichtung zur Bestimmung der Berstfestigkeit, des Elastizitätsmoduls und der mechanischen Verformungsarbeit an Proben (**1**) essbarer Membranen nach Anspruch 1, **dadurch gekennzeichnet, dass** diese aus einem Edelstahlbehälter (**2**), zwei Ringen (**3**) und (**5**) aus Edelstahl, zwei Dichtungen(**4**) und (**6**) aus Silikongummi, zwei Kippschrauben (**7**) und (**8**), zwei Rändelmuttern (**9**) und (**10**), einer Schraube (**11**), einem Gusseisensupport (**12**), einer zylindrische Stahlsäule (**13**), einer Rändelschraube (**14**), einer Stahltraverse (**15**) einer Schwalbenschwanz Führung(**16**) und (**17**), einer Schraube(**18**) mit gerändeltem Kopf, einem berührungslosen Bewegungssensor (**19**) mit konfokaler Laser Triangulation, einem elektronisches Manometer (**20**) mit USB-Schnittstelle und die Speicherung des maximalen Luftdruckwertes, einer pneumatischen Einheit (**21**) zur linearen Erhöhung des Luftdrucks, einer pneumatischen Einheit (**22**) zur Sicherung eines konstanten Drucks, einem Computer (**23**), einem Drucker (**24**) und einer Software zur Programmierung der Druckerhöhung im Behälter (**2**) sowie zur Datenverarbeitung, gebildet ist.

FIG.1

FIG.2

FIG. 3

*FIG.7*

*FIG.4*

**FIG.5**

**SCHNITT A-A**

**FIG.6**

FIG. 8

Europäisches
Patentamt

European
Patent Office

Office européen
des brevets

**EUROPÄISCHER RECHERCHENBERICHT**

**Nummer der Anmeldung**

**EP 21 00 0204**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 1 443 317 A2 (UNITED BISCUITS LTD [GB]) 4. August 2004 (2004-08-04) * Zusammenfassung; Abbildungen 1-9 * * Absätze [0027], [0083], [0084], [0098] * ----- | 1-6 | INV. G01N3/04 G01N3/06 G01N3/12 G01N33/02 G01N3/20 G01M5/00 |
| X | CN 205 898 575 U (UNIV JINAN) 18. Januar 2017 (2017-01-18) * Absätze [0002], [0006], [0008], [0013], [0015]; Anspruch 6; Abbildung 1 * ----- | 1-6 | |
| X | US 6 050 138 A (LYNCH KEVIN R [US] ET AL) 18. April 2000 (2000-04-18) * Zusammenfassung; Abbildung 5 * ----- | 1-6 | |

**RECHERCHIERTE SACHGEBIETE (IPC)**

G01N
G01M

Der vorliegende Recherchenbericht wurde für alle Patentansprüche erstellt

| Recherchenort | Abschlußdatum der Recherche | Prüfer |
|---|---|---|
| **Den Haag** | **12. Januar 2022** | **Giráldez Sánchez, J** |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument

......................................................................................................
& : Mitglied der gleichen Patentfamilie, übereinstimmendes
    Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

**EP 21 00 0204**

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

**12-01-2022**

| Im Recherchenbericht angeführtes Patentdokument | Datum der Veröffentlichung | Mitglied(er) der Patentfamilie | Datum der Veröffentlichung |
|---|---|---|---|
| EP 1443317 A2 | 04-08-2004 | AT 408811 T<br>EP 1443317 A2<br>GB 2399183 A | 15-10-2008<br>04-08-2004<br>08-09-2004 |
| CN 205898575 U | 18-01-2017 | KEINE | |
| US 6050138 A | 18-04-2000 | AU 1190899 A<br>CA 2308799 A1<br>EP 1025420 A2<br>US 6050138 A<br>US 6321594 B1<br>US 6349588 B1<br>WO 9921057 A2 | 10-05-1999<br>29-04-1999<br>09-08-2000<br>18-04-2000<br>27-11-2001<br>26-02-2002<br>29-04-1999 |

Für nähere Einzelheiten zu diesem Anhang : siehe Amtsblatt des Europäischen Patentamts, Nr.12/82

EPO FORM P0461